# EUROPÄISCHE PATENTSCHRIFT

(11) **EP 0 432 636 B1**
(45) Veröffentlichungstag und Bekanntmachung des Hinweises auf die Patenterteilung: **27.10.1993**
(21) Anmeldenummer: 90123399.9
(22) Anmeldetag: 06.12.1990
(51) Int. Cl.: C07C 21/10, C07C 17/00

(54) **Verfahren zur Herstellung von Trichlorethylen**
Process for preparing trichloroethylene
Procédé de préparation du trichloréthylène

(30) Priorität: 12.12.1989 DE 3941037
(43) Veröffentlichungstag der Anmeldung: 19.06.1991
(73) Patentinhaber: WACKER-CHEMIE GMBH, D-81737 München (DE)
(72) Erfinder: Dafinger, Willibald, Dr., W-8261 Emmerting (DE); Gabler, Wolfdietrich, Dr., W-8263 Burghausen (DE); Pichl, Eduard, W-8261 Mehring (DE); Hierzegger, Roman, W-8263 Burghausen (DE)

(56) Entgegenhaltungen:
- DE-A- 2 819 209
- DE-A- 3 804 265
- DE-B- 1 194 403

## Beschreibung

Die Erfindung betrifft ein Verfahren zur Herstellung von Trichlorethylen aus Perchlorethylen und Wasserstoff mittels eines Kupfer/Rhodium-Katalysators, der mit einem wasserlöslichen Phosphoniumhalogenid imprägniert ist.

Aus der DE-A 2819209 (EP-B 5263) ist ein Verfahren bekannt, Trichlorethylen aus Perchlorethylen und Wasserstoff herzustellen, wobei ein Katalysator eingesetzt wird, bestehend aus einem Aktivkohleträger, imprägniert mit Kupfer in elementarer oder chemisch gebundener Form, sowie Palladium, Rhodium oder Ruthenium, jeweils in elementarer oder chemisch gebundener Form. Nachteilig bei dieser Verfahrensweise sind die hohen Temperaturen, die zum Erhalt von befriedigenden Umsatzraten erforderlich sind.

Zur Erhöhung der Katalysatoraktivität bei niederer Reaktionstemperatur wird in der DE-A 3804265 vorgeschlagen, mit Phosphinen oder Phosphiten imprägnierte Kupfer/Rhodium-Katalysatoren einzusetzen. Nachteilig bei dieser Verfahrensweise ist der Umstand, daß zur Imprägnierung des Katalysatorträgers in einem aufwendigen Zweistufen-Verfahren zunächst die wäßrige Lösung der Kupfer- bzw. Rhodiumverbindung, im folgenden Schritt dann die in organischen Lösemitteln gelösten Phosphine oder Phosphite aufgetragen werden müssen. Vor allem aber ist die Steigerung der Katalysatoraktivität zeitlich begrenzt, so daß schon bei Betriebszeiten unter 1000 Stunden dem Aktivitätsabfall mit einer Erhöhung der Betriebstemperatur entgegengewirkt werden muß.

Es bestand daher die Aufgabe, ein Verfahren zur Herstellung von Trichlorethylen aus Perchlorethylen und Wasserstoff zur Verfügung zu stellen mit dem bereits bei niederer Betriebstemperatur auch bei langen Betriebszeiten hohe Umsätze erreicht werden können.

Gegenstand der Erfindung ist ein Verfahren zur Herstellung von Trichlorethylen aus Perchlorethylen und Wasserstoff mittels eines Trägerkatalysators, bestehend aus Aktivkohle mit mehr als 500 m²/g BET-Oberfläche, 0.5 bis 20 Gew.% Kupfer in elementarer oder chemisch gebundener Form und 0.01 bis 1.0 Gew.% Rhodium oder Palladium in elementarer oder chemisch gebundener Form bei einer Temperatur von 150 bis 250°C und einem Wasserstoffdruck von 1 bis 10 bar absolut, dadurch gekennzeichnet, daß der Trägerkatalysator mit 0.1 bis 10.0 Gew.% eines wasserlöslichen Phosphoniumhalogenids imprägniert ist.

Als Katalysatorträger wird Aktivkohle, vorzugsweise in gekörnter Form, mit einer BET-Oberfläche von mehr als 500 m²/g und einer Korngröße von 2 bis 10 mm eingesetzt.

Das Kupfer wird zu 0.5 bis 20.0 Gew.%, vorzugsweise zu 5.0 bis 15.0 Gew.%, bezogen auf das Gesamtgewicht aus Katalysatorträger und Aktivkomponenten, in elementarer oder chemisch gebundener Form auf den Träger aufgetragen. Besonders bevorzugt werden wasserlösliche Kupfersalze, insbesonders CuCl₂, eingesetzt.

Rhodium wird zu 0.01 bis 1.0 Gew.%, vorzugsweise zu 0.02 bis 0.2 Gew.%, bezogen auf das Gesamtgewicht aus Katalysatorträger und Aktivkomponenten, in elementarer oder chemisch gebundener Form eingesetzt. Besonders bevorzugt werden wasserlösliche Rhodiumverbindungen, insbesonders Komplexsalze des Rhodium(III)-chlorids. Statt Rhodium kann auch Palladium in elementarer oder chemisch gebundener Form und in den eben für Rhodium angegebenen Mengen eingesetzt werden. Besonders bevorzugt sind auch hier wasserlösliche Palladiumverbindungen, wie etwa PdCl₂.

Der Katalysatorträger ist erfindungsgemäß noch mit 0.1 bis 10.0 Gew.%, vorzugsweise 3.0 bis 7.0 Gew.%, bezogen auf das Gesamtgewicht aus Katalysatorträger und Aktivkomponenten, eines wasserlöslichen Phosphoniumhalogenids imprägniert. Vorzugsweise werden wasserlösliche Phosphoniumhalogenide der allgemeinen Formel (Ph₃PR)X eingesetzt; wobei Ph für einen Phenylrest steht.

R steht für Wasserstoff oder einen, gegebenenfalls substituierten Alkyl- oder Arylrest.
Beispiele hierfür sind Methyl-, Ethyl-, Propyl-, n-Butyl-, iso-Butyl-, Pentyl-, Hexyl-, Heptyl-, Octyl-, Nonyl-, Benzyl-, p-Chlorbenzyl-, p-tert.-Butylbenzyl-, Allyl-, 2-Methallyl, Chlormethyl-, Dichlormethyl-, Iodmethyl-, Ethoxycarbonylmethyl- oder Acetonyl-Rest.

Als Halogenid X werden Chlorid, Iodid oder Bromid bevorzugt eingesetzt.

Bevorzugte Phosphoniumhalogenide sind Methyltriphenylphosphoniumchlorid, Methyltriphenylphosphoniumbromid, Ethyltriphenylphosphoniumchlorid, Ethyltriphenylphosphoniumbromid, n-Propyltriphenylphosphoniumchlorid, n-Propyltriphenylphosphoniumbromid, Allyltriphenylphosphoniumchlorid, Allyltriphenylphosphoniumbromid, n-Butyltriphenylphosphoniumchlorid und n-Butyltriphenylphosphoniumbromid.

Besonders bevorzugt sind Methyltriphenylphosphoniumchlorid, Methyltriphenylphosphoniumbromid, Ethyltriphenylphosphoniumchlorid und Ethyltriphenylphosphoniumbromid.

Die Herstellung der Phosphoniumhalogenide kann in an sich bekannter Art und Weise erfolgen. Beispielsweise erhält man durch Umsetzen von Triphenylphosphin mit den entsprechenden Halogenwasserstoffverbindungen die Triphenylphosphoniumsalze. Setzt man die gegebenenfalls substituierten Alkylhalogenide ein, werden die Alkyl-triphenylphosphoniumsalze zugänglich. Weitere Verfahrensweisen zur Herstellung von Phosphoniumhalogeniden sind in folgenden Publikationen beschrieben:
J. Buddrus, Chem. Ber. 107, 2062 (1974); G. Wittig, U. Schöllkopf, Chem. Ber. 87, 1318 (1954); D. Denney, L. Smith, J. Org. Chem. 45, 3404 (1962); G. Wittig, M. Schlosser, Chem. Ber. 94, 1373 (1961); G. Aksnes, A. Eide, Phosphorus 4 (3), 209 (1974); Houben-Weyl, Methoden der organischen Chemie 12/1, 79 (1963); Ullmann, Encyclopädie der technischen Chemie 18, 380 (1979).

Zur Imprägnierung werden die einzelnen Komponenten, Kupfer(salz), Rhodium(salz) oder Palladium(salz), und Phosphoniumverbindung vorzugsweise in wäßriger Lösung, getrennt oder im Gemisch, auf die Aktivkohle, zum Beispiel durch Tränken, aufgetragen. Anschließend wird der so imprägnierte Katalysatorträger getrocknet.

Zur Umsetzung des Perchlorethylens wird der Katalysator in geschütteter Form in ein Reaktionsrohr gebracht. Die Reaktion wird bei einer Temperatur von 150 bis 250°C und unter einem Druck von 1 bis 10 bar absolut durchgeführt. Das Perchlorethylen wird vorzugsweise in Mengen von 0.5 bis 5.0 Mol pro Stunde und pro Liter an Kontaktmasse (Katalysatorvolumen) zusammen mit der 0.1- bis 1-fachen molaren Menge pro Stunde an Wasserstoff zur Reaktion gebracht.

Die folgenden Beispiele dienen zur weiteren Erläuterung der Erfindung:

### Beispiel 1:

Gekörnte Aktivkohle mit einer BET-Oberfläche von 800 m²/g und einer Korngröße von 3 mm (Degusorb WS IV Spezial, Fa. Degussa) wurde mit wäßrigen Lösungen von CuCl₂, Na₃RhCl₆ und [(C₆H₅)₃PCH₃]Cl getränkt und anschließend getrocknet, so daß der Cu-Gehalt der Aktivkohle 10 Gew.% der Rh-Gehalt 0.044 Gew.% und der [C₆H₅)PCH₃]Cl-Gehalt 6.5 Gew.% betragen hat. Die Trichlorethylenherstellung wurde in einem Einrohrreaktor mit einem Katalysatorvolumen von 1500 ml durchgeführt. Der Reaktor wurde mit 960 g/h (5.8 mol/h) Perchlorethylen und 70 l/h (3.5 mol/h) Wasserstoff belastet. Der Reaktionsdruck betrug 6 bar absolut.

### Vergleichsbeispiel 1:

Es wurde analog Beispiel 1 vorgegangen. Anstelle des Methyltriphenylphosphoniumchlorids wurde die äquivalente Menge Triphenylphosphin eingesetzt.

### Vergleichsbeispiel 2:

Es wurde analog Beispiel 1 vorgegangen; jedoch ohne Zusatz von Methyltriphenylphosphoniumchlorid.

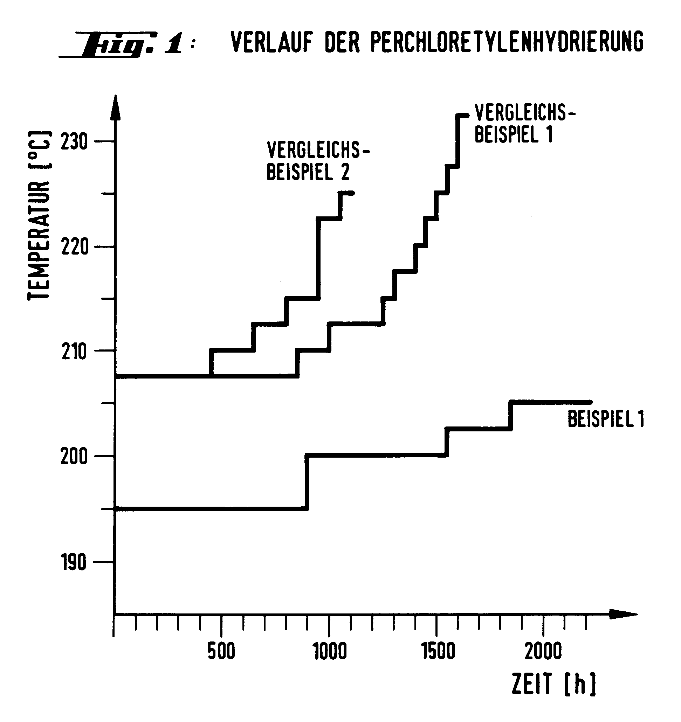

Die Ergebnisse von Beispiel 1 bzw. Vergleichsbeispiel 1 und Vergleichsbeispiel 2 sind in Figur 1 zusammengefaßt. Figur 1 zeigt den Temperatur-Zeit-Verlauf (T/t-Verlauf) der Perchlorethylenhydrierung gemäß den obigen Beispielen bei einer Umsatzrate zu Trichlorethylen von 40 Gew.%

### Beispiel 2:

Es wurde analog Beispiel 1 vorgegangen. Als phosphoniumhalogenid wurde allerdings Methyltriphenylphosphoniumbromid eingesetzt. Die Reaktion sprang ebenfalls bei 195°C an. Wie in Beispiel 1 blieb die Umsatzrate bei dieser Temperatur über einen Zeitraum von 900 Stunden bei 40 Gew.%.

### Beispiel 3:

Es wurde analog Beispiel 1 vorgegangen. Als phosphoniumhalogenid wurde allerdings Ethyltriphenylphosphoniumchlorid eingesetzt. Die Reaktion sprang ebenfalls bei 195°C an. Wie in Beispiel 1 blieb die Umsatzrate bei dieser Temperatur über einen Zeitraum von 900 Stunden bei 40 Gew.%.

### Beispiel 4:

Es wurde analog Beispiel 1 vorgegangen. Als phosphoniumhalogenid wurde allerdings Ethyltriphenylphosphoniumbromid eingesetzt. Die Reaktion sprang ebenfalls bei 195°C an. Wie in Beispiel 1 blieb die Umsatzrate bei dieser Temperatur über einen Zeitraum von 900 Stunden bei 40 Gew.%.

### Beispiel 5:

Es wurde analog Beispiel 1 vorgegangen. Als phosphoniumhalogenid wurde allerdings n-Propyltriphenylphosphoniumchlorid eingesetzt. Die Reaktion sprang ebenfalls bei 195°C an. Wie in Beispiel 1 blieb die Umsatzrate bei dieser Temperatur über einen Zeitraum von 900 Stunden bei 40 Gew.%.

### Beispiel 6:

Es wurde analog Beispiel 1 vorgegangen. Als Phosphoniumhalogenid wurde allerdings n-Propyltriphenylphosphoniumbromid eingesetzt. Die Reaktion sprang ebenfalls bei 195°C an. Wie in Beispiel 1 blieb die Umsatzrate bei dieser Temperatur über einen Zeitraum von 900 Stunden bei 40 Gew.%.

### Beispiel 7:

Es wurde analog Beispiel 1 vorgegangen. Als phosphoniumhalogenid wurde allerdings Allyltriphenylphosphoniumchlorid eingesetzt. Die Reaktion sprang ebenfalls bei 195°C an. Wie in Beispiel 1 blieb die Umsatzrate bei dieser Temperatur über einen Zeitraum von 900 Stunden bei 40 Gew.%.

### Beispiel 8:

Es wurde analog Beispiel 1 vorgegangen. Als Phosphoniumhalogenid wurde allerdings Allyltriphenylphosphoniumbromid eingesetzt. Die Reaktion sprang ebenfalls bei 195°C an. Wie in Beispiel 1 blieb die Umsatzrate bei dieser Temperatur über einen Zeitraum von 900 Stunden bei 40 Gew.%.

### Beispiel 9:

Es wurde analog Beispiel 1 vorgegangen. Als Phosphoniumhalogenid wurde allerdings n-Buyltriphenylphosphoniumchlorid eingesetzt. Die Reaktion sprang ebenfalls bei 195°C an. Wie in Beispiel 1 blieb die Umsatzrate bei dieser Temperatur über einen Zeitraum von 900 Stunden bei 40 Gew.%.

### Beispiel 10:

Es wurde analog Beispiel 1 vorgegangen. Als Phosphoniumhalogenid wurde allerdings n-Butyltriphenylphosphoniumbromid eingesetzt. Die Reaktion sprang ebenfalls bei 195°C an. Wie in Beispiel 1 blieb die Umsatzrate bei dieser Temperatur über einen Zeitraum von 900 Stunden bei 40 Gew.%.

## Patentansprüche

1. Verfahren zur Herstellung von Trichlorethylen aus Perchlorethylen und Wasserstoff mittels eines Trägerkatalysators, bestehend aus Aktivkohle mit mehr als 500 m²/g BET-Oberfläche, 0.5 bis 20 Gew.% Kupfer in elementarer oder chemisch gebundener Form und 0.01 bis 1.0 Gew.% Rhodium oder Palladium in elementarer oder chemisch gebundener Form bei einer Temperatur von 150 bis 250°C und einem Wasserstoffdruck von 1 bis 10 bar absolut, dadurch gekennzeichnet, daß der Trägerkatalysator mit 0.1 bis 10.0 Gew.% eines wasserlöslichen Phosphoniumhalogenids imprägniert ist.

2. Verfahren nach Anspruch 1, dadurch gekennzeichnet, daß eine Phosphoniumverbindung der allgemeinen Formel (Ph₃PR)X eingesetzt wird, wobei Ph für einen Phenylrest steht; R für Wasserstoff oder einen substituierten oder unsubstituierten Alkyl- oder Arylrest; und X für Chlorid, Bromid oder Iodid steht.

3. Verfahren nach Anspruch 2 , dadurch gekennzeichnet, daß R für einen Methyl- oder Ethylrest steht.

4. Verfahren nach Anspruch 1, dadurch gekennzeichnet, daß als wasserlösliches Phosphoniumhalogenid Methyltriphenylphosphoniumchlorid, Methyltriphenylphosphoniumbromid, Ethyltriphenylphosphoniumchlorid oder Ethyltriphenylphosphoniumbromid eingesetzt wird.

## Claims

1. Process for the preparation of trichloroethylene from perchloroethylene and hydrogen by means of a supported catalyst consisting of active charcoal having a BET surface area of more than 500 m²/g, 0.5 to 20% by weight of copper in elemental or chemically bonded form and 0.01 to 1.0% by weight of rhodium or palladium in elemental or chemically bonded form at a temperature of 150 to 250°C and a hydrogen pressure of 1 to 10 bar absolute, which comprises impregnating the supported catalyst with 0.1 to 10.0% by weight of a water-soluble phosphonium halide.

2. Process according to Claim 1, characterised in that a phosphonium compound of the general formula (Ph₃PR)X, in which Ph represents a phenyl radical; R for [sic] hydrogen or a substituted or unsubstituted alkyl or aryl radical; and X represents chloride, bromide or iodide, is employed.

3. Process according to Claim 2, characterised in that R represents a methyl or ethyl radical.

4. Process according to Claim 1, characterised in that methyltriphenylphosphonium chloride, methytriphenylphosphonium bromide, ethyltriphenylphosphonium chloride or ethyltriphenylphosphonium bromide is employed as the water-soluble phosphonium halide.

## Revendications

1. Procédé de préparation de trichloréthylène à partir de perchloréthylène et d'hydrogène avec un catalyseur sur support formé de charbon actif ayant une surface BET supérieure à 500 m²/g et portant de 0,5 à 20% en poids de cuivre à l'état élémentaire ou en liaison chimique et de 0,01 à 1,0% de rhodium ou de palladium, également élémentaire ou en liaison chimique, à une température de 150 à 250°C et sous une pression absolue d'hydrogène de 1 à 10 bar, procédé caractérisé en ce que le catalyseur sur support est imprégné de 0,1 à 10% en poids d'un halogénure de phosphonium hydrosoluble.

2. Procédé selon la revendication 1, caractérisée en ce que l'on utilise un composé de phosphonium de formule (Ph₃PR)X, Ph désignant le radical phényle, R l'hydrogène ou un radical alkyle ou aryle avec ou sans substituants et X le chlore, le brome ou l'iode.

3. Procédé selon la revendication 2, caractérisé en ce que dans la formule indiquée R est le radical méthyle ou éthyle.

4. Procédé selon la revendication 1, caractérisé en ce que l'on prend comme halogénure de phosphonium hydrosoluble le chlorure ou le bromure de méthytriphénylphosphonium ou le chlorure ou le bromure d'éthyltriphénylphosphonium.
